# EUROPÄISCHE PATENTSCHRIFT

(11) **EP 2 145 585 B1**
(45) Veröffentlichungstag und Bekanntmachung des Hinweises auf die Patenterteilung: **07.09.2016**
(21) Anmeldenummer: 09009259.4
(22) Anmeldetag: 16.07.2009
(51) Int. Cl.: A61B 17/00

(54) **Punktionsverschluss zum Verschließen eines eine Punktionsöffnung aufweisenden Hohlorgans, insbesondere eines Blutgefäßes**
Puncture seal for sealing a hollow organ with a puncture opening, in particular a blood vessel
Fermeture de ponction destiné à fermer un organe creux comprenant une ouverture de ponction, notamment un vaisseau sanguin

(30) Priorität: 18.07.2008 DE 102008034534
(43) Veröffentlichungstag der Anmeldung: 20.01.2010
(73) Patentinhaber: Aesculap AG, 78532 Tuttlingen/Donau (DE)
(72) Erfinder: Inderbitzi, Rolf, 8967 Widen (CH)
(74) Vertreter: Patentanwälte Ruff, Wilhelm, Beier, Dauster & Partner mbB

(56) Entgegenhaltungen:
- US-A- 5 350 399
- US-A1- 2003 120 291
- US-A1- 2005 033 361
- US-A1- 2005 187 564
- US-A1- 2006 212 047

## Beschreibung

Die Erfindung betrifft einen Punktionsverschluss zum Verschließen eines eine Punktionsöffnung in einer Wandung aufweisenden Hohlorgans, insbesondere eines Blutgefäßes, umfassend ein inneres Anlageelement zur Anlage an die intraluminale Seite der Wandung des Hohlorgans und ein mit dem inneren Anlageelement verbundenes Verbindungselement, das im montierten Zustand des Verschlusses durch die Punktionsöffnung hindurchragt, wobei das innere Anlageelement eine Flächenausdehnung besitzt, die größer als die Punktionsöffnung ist.

In der Angiologie, Kardiologie, interventionellen Radiologie und Neuroradiologie sowie auf weiteren Gebieten, beispielsweise der Chirurgie, werden perkutane, transluminale Kathetereingriffe in Hohlorgane vorgenommen. In der Regel wird für diese Eingriffe als Zugang die Arteria femoralis communis in der Leiste benützt. Am Ende des Eingriffs verbleibt eine geöffnete Arterie. In der Arterie pulsiert Blut mit einem systolischen Druck von mehr als 100 mm Hg. Wird die Arterienöffnung nicht verschlossen, besteht die Gefahr, dass sich eine Blutung entwickelt, welche als Hämatom und/oder als Aneurysma spurium klinische Bedeutung erlangt, d.h. behandelt werden muss. Um diesen Komplikationen vorzubeugen, wird am Ende der Intervention der operative Zugang entweder mittels Kompression oder durch Verwendung eines Verschlusssystems abgedichtet. Ist der für die Intervention benötigte Schleusendurchmesser größer als 5 Charriere, werden zur Abdichtung der Arterienöffnung routinemäßig Verschlusssysteme eingesetzt. Zunehmend werden auch kathetertechnische Interventionen durchgeführt, für welche großlumige Schleusen benötigt werden. Deren Durchmesser erreicht zum Beispiel für das Einbringen einer Aorto-iliakalen-Endoprothese 28 Charriere.

Die Veröffentlichungen Arterial closure devices von J.B. Madigan, L.A. Ratnam und A.M. Belli in The Journal of cardiovascular surgery, 2007; 48:607-624 enthält eine anschauliche Zusammenfassung der bisher praktisch verwendeten Verschlusssysteme. In dieser Veröffentlichung beschriebene und weitere Verschlusssysteme sind aus der folgenden Patentliteratur bekannt: US 4,744,364, US 5,350,399, US 5,441,517, US 5,593,422, US 5,620,461, US 5,916,236, US 6,764,500 B1 (entsprechend EP 0 474 752 B1), US 7,169,168 B2, US 2007/0135842 A1, WO 89/11301, EP 0 776 179 B1, DE 196 20 620 A1, EP 0 955 901 B1 und EP 0 474 752 B2.

Die US 2006/0212047 A1 offenbart eine wendelförmig ausgebildete Verschlussvorrichtung für Septumdefekte. Gegenstand der US 2005/0033361 A1 ist eine wendelförmig ausgebildete Verschlussvorrichtung, welche im montierten Zustand eine konische bzw. pilzförmige Konfiguration besitzt. Die US 2005/0187564 A1 offenbart eine gewendelte Verschlussvorrichtung mit einem inneren und einem äußeren Anlageelement, wobei die Anlageelemente im montierten Zustand ebenfalls konisch bzw. pilzförmig ausgebildet sind. Aus der US 2003/0120291 A1 geht eine Verschlussvorrichtung hervor, welche in montiertem Zustand ein konisches bzw. ein pilzförmig ausgebildetes inneres Anlageelement aufweist. Die US 5,350,399 bezieht sich auf eine Verschlussvorrichtung mit einem entfaltbaren inneren Anlageelement und einem entfaltbaren äußeren Anlageelement, wobei die Anlageelemente nach ihrer Entfaltung eine regenschirmartige Konfiguration besitzen.

In der Regel wird bei den bekannten Verschlusssystemen ein inneres Anlageelement, das zur Anlage an der Innenwand des Hohlorgans im Bereich der Punktionsöffnung bestimmt ist, in gefaltetem oder aufgerolltem Zustand durch einen Katheter in das Innere des Hohlorgans eingeführt und danach entfaltet. Das innere Anlageelement ist dabei in der Regel mit einem Halte- bzw. Verbindungselement versehen, das durch die Punktionsöffnung in der Wandung des Hohlorgans und durch die darüber liegende Haut hindurchgeführt ist und dort festgelegt ist, um das Anlageelement an der richtigen Stelle zu halten. Als Halte- bzw. Verbindungselement dient in der Regel eine Schnur oder ein Stab. Bekannt sind auch äußere Anlageelemente, die auf der Außenseite der Wandung des Hohlorgans platziert werden und mit dem inneren Anlageelement über das Verbindungselement verbunden sind.

Das Einbringen des inneren Anlageelementes und seine Entfaltung können zu Komplikationen führen, insbesondere dann, wenn der Durchmesser des Hohlorgans gering ist. Bei manchen Verschlusssystemen wird nicht nur bei der Montage sondern auch danach der Durchflussquerschnitt des Hohlorgans in einer nicht akzeptierbaren Weise verstellt.

Der Erfindung liegt die Aufgabe zugrunde, ein Verschlusssystem zu schaffen, bei dem das innere Anlageelement im Wesentlichen unabhängig vom Durchmesser des Hohlorgans in einfacher Weise ohne Störungen des Durchflusses in das Hohlorgan eingebracht werden kann. Die Erfindung ist ausgehend von dem oben beschriebenen Verschlusssystem dadurch gekennzeichnet, dass das innere Anlageelement als zusammenhängender bogenförmig verlaufender Streifen ausgebildet ist, wobei Streifenabschnitte im montierten Zustand flach nebeneinander liegen und die Streifenabschnitte eine Breite haben, die kleiner ist als die größte Länge der Punktionsöffnung, und das innere Anlageelement entlang des Streifens im Wesentlichen parallel zur Wandung des Hohlorgans in dieses einführbar ist.

Bei einer Arterie, die in den meisten Fällen das Hohlorgan ist, ist die Punktionsöffnung in der Regel nicht kreisrund sondern schlitzförmig, wobei der Schlitz in der Wandung der Arterie in der Regel quer zur Längsrichtung der Arterie verläuft. Diese Ausbildung der Punktionsöffnung ist bedingt durch die Faserrichtung in der Wandung der Arterie.

Gemäß der Erfindung ist es nicht wie beim Stand der Technik erforderlich, das innere Anlageelement in der Flächenausdehnung klein zu machen, d.h. zusammenzufalten oder aufzurollen, um es durch die Punktionsöffnung transportieren zu können. Demgegenüber ist es bei der Erfindung vorgesehen, das normalerweise flächig ausgebildete Anlageelement derart linienförmig zu unterteilen, dass sich aus der Fläche des Anlageelementes ein zusammenhängender Streifen ergibt, dessen Breite geringer ist als die größte Länge der Punktionsöffnung. Dies ermöglicht es, das Anlageelement als Streifen durch den Schlitz der Punktionsöffnung in das Innere des Hohlorgans einzuführen und zwar im Wesentlichen parallel zur Wandung des Hohlorgans. Dadurch wird eine Beeinträchtigung des Querschnittes des Hohlorgans und insbesondere eine Beschädigung der gegenüber liegenden Wandungsinnenseite vermieden.

Die Länge des Streifens kann ein Vielfaches der größten Länge der Punktionsöffnung betragen, in der Regel mindestens das 5-fache, vorzugsweise mindestens das 10-fache. Die Flächenausdehnung des inneren Anlageelementes ist in den Flächendimensionen, d.h. Länge und Breite bzw. Durchmesser, vorzugsweise größer als die Breite der Streifenabschnitte des Streifens. In der Regel beträgt die Flächendimension mindestens das 3-fache der Breite eines Streifens, vorzugsweise ca. das 5-fache. Dies bedeutet, dass mindestens drei, vorzugsweise ca. fünf, Streifenabschnitte nebeneinander liegen.

Bei einer bevorzugten Ausführungsform der Erfindung ist der Streifen zumindest im montierten Zustand flach und verläuft in der Fläche des inneren Anlageelementes bogenförmig. Dies bedeutet, dass der Streifen zumindest im montierten Zustand die Form einnimmt, die ein flächiges inneres Anlageelement normalerweise hat. Im nicht montierten Zustand kann der Streifen nicht nur in der Fläche gebogen sein sondern auch senkrecht dazu, worauf nachfolgend näher eingegangen wird. Vorzugsweise ist der Streifen derartig bogenförmig ausgebildet, dass sich benachbarte Streifenabschnitte bilden. Dies bedeutet, dass die Streifenabschnitte vorzugsweise so nahe nebeneinander liegen, dass sich wiederum eine Gesamtfläche ergibt. Vorzugsweise ist das innere Anlageelement zumindest im montierten Zustand scheibenförmig ausgebildet. Die Scheibe ist bei einer Ausführungsform vorzugsweise im Wesentlichen kreisrund. Der Streifen kann spiralig gebogen verlaufen, wobei sich eine im Wesentlichen kreisrunde Scheibenform ergibt. So hat der Streifen vorzugsweise die Form einer flächigen Spirale. Bei einer bevorzugten Ausführungsform der Erfindung hat der Streifen die Form einer spiraligen Wendel bzw. wendelförmigen Spirale. Dies bedeutet, dass der flächige Streifen spiralig gebogen ist und die einzelnen Windungen der Spirale gleichzeitig eine wendelförmige Steigung besitzen. Bevorzugt besteht der Streifen aus einem mindestens zum Teil elastischen Material. So ist bei einer besonders bevorzugten Ausführungsform das innere Anlageelement in einem ersten Funktionszustand (vor der fertigen Montage) als Wendelspirale ausgebildet und in einem zweiten Funktionszustand (nach Montage) plattenförmig bzw. scheibenförmig mit einer im Wesentlichen kreisrunden Anlagefläche. Bevorzugt ist das innere Anlageelement mit Hilfe des Verbindungselementes durch Zug an diesem von dem ersten Funktionszustand in den zweiten Funktionszustand überführbar.

Bei einer anderen Ausführungsform der Erfindung ist der Streifen im Wesentlichen schlangenförmig bzw. mäanderförmig gebogen. Hier liegt der Streifen normalerweise in der flächigen Ebene. Es ist aber auch hier möglich, dem Streifen eine gewisse Steigung aus der Ebene zu verleihen. Im montierten Zustand liegt er dann mit einer gewissen Vorspannung an der Gefäßwandung an.

Es ist bevorzugt, dass benachbarte Streifenabschnitte einen Abstand voneinander haben oder in einen solchen bringbar sind, der zumindest der Wandungsstärke des Hohlorgans entspricht. Dies bedeutet für die Praxis, dass dieser Abstand mindestens 1 mm, in der Regel 1 bis 2 mm, beträgt, da die Wandstärke einer Arterie im Leistenbereich ungefähr 1 mm beträgt. Der Abstand der benachbarten Streifenabschnitte kann in der Fläche des inneren Anlageelementes und/oder senkrecht dazu liegen. So kann in der Fläche zwischen den Streifenabschnitten ein lichter Zwischenraum bestehen. Ein Zwischenraum kann auch durch eine Steigung der Streifenabschnitte erzeugt werden. Ferner ist eine Kombination beider Arten möglich. So kann der Abstand zwischen den benachbarten Streifenabschnitten durch elastische Verschiebung der Streifenabschnitte schräg, vorzugsweise senkrecht, zur Fläche des inneren Anlageelementes erzeugbar oder erweiterbar sein. Andererseits können benachbarte Streifenabschnitte infolge unterschiedlicher Steigung einen Abstand haben, der durch elastische Kompression senkrecht zur Fläche des Anlageelementes verkleinerbar oder beseitigbar ist. Diese Möglichkeit ist besonders von Vorteil bei einer Ausführungsform, bei der sich benachbarte Streifenabschnitte im montierten Zustand gegenseitig überlappen. Durch eine solche Überlappung kann eine erhöhte Stabilisierung des streifenförmig ausgebildeten Anlageelementes erhalten werden. Eine solche Überlappung kann ausgebildet sein, indem die Trennlinien zwischen den Streifenabschnitten schräg zur Fläche des inneren Anlageelementes verlaufen. Auch ein gegenseitiges Ineinandergreifen der Streifenabschnitte an ihren Rändern ist möglich. So können benachbarte Streifenabschnitte im montierten Zustand eine im Wesentlichen geschlossene Fläche bilden.

Das Verbindungselement dient dazu, das innere Anlageelement zu positionieren und an der richtigen Stelle zu halten sowie eine Verbindung vom inneren Anlageelement nach außerhalb des Hohlorgans zu schaffen. Es kann flexibel sein, beispielsweise aus einem Draht oder einer Schnur bestehen. Vorzugsweise ist es starr ausgebildet. Insbesondere dann, wenn es starr ist, ist es mit Vorteil drehfest mit dem inneren Anlageelement verbunden. Vorzugsweise ist das Verbindungselement einstückig mit dem inneren Anlageelement ausgebildet. Bei einer besonders bevorzugten Ausführungsform der Erfindung ist dem inneren Anlageelement ein äußeres Gegenlager zugeordnet, insbesondere ein äußeres Anlageelement, das zur Anlage an die extraluminale Seite des Hohlorgans bestimmt ist. Das Gegenlager, insbesondere das äußere Anlageelement, ist vorzugsweise auf dem Verbindungselement festlegbar. Weiterhin kann das Gegenlager, insbesondere das äußere Anlageelement, eine mittige Öffnung mit einem lichten Durchmesser aufweisen, der im wesentlichen dem Außendurchmesser des Verbindungselementes entspricht. Das innere Anlageelement und das äußere Gegenlager können mit Hilfe des Verbindungselementes im montierten Zustand miteinander verbunden sein. Durch die Verbindung kann, wenn gewünscht, ein gewisser Anlagedruck zwischen innerem Anlageelement und Gegenlager auf die abzudichtende Wandung erzeugt werden.

Bei einer Ausführungsform der Erfindung ist das innere Anlageelement mit einer zugfesten Applikationshilfe verbindbar oder verbunden. Eine solche Applikationshilfe kann mit dem Verbindungselement kombiniert sein. Die Applikationshilfe ist vorzugsweise rohrförmig ausgebildet, insbesondere nach Art einer Drehhülse. Die Applikationshilfe kann weiterhin flexibel sein und beispielsweise von einer Schnur oder einem Draht gebildet werden. Die Applikationshilfe kann gleichzeitig als Verbindungselement dienen. Die Applikationshilfe kann bei einer anderen Ausführungsform von einer Verlängerung eines im Wesentlichen starren Verbindungselementes gebildet werden. Nach dem Anbringen des Punktionsverschlusses nicht mehr benötigte Stücke des Verbindungselementes können gekürzt werden. Es können auch getrennte Applikationshilfen vorgesehen sein, die mit dem inneren Anlageelement in Wirkverbindung gebracht werden können. Ihr Angriff erfolgt vorzugsweise mittelbar über das Verbindungselement oder über ein Gegenlager. Bei einer besonders bevorzugten Ausführungsform der Erfindung weist das äußere Anlageelement eine mittige Öffnung auf, die einen lichten Durchmesser besitzt, der im Wesentlichen dem Außendurchmesser des Verbindungselementes entspricht. Das äußere Anlageelement ist somit auf das Verbindungselement aufschiebbar. Vorzugsweise können Rastelemente vorgesehen sein, mit deren Hilfe das äußere Anlageelement auf dem Verbindungselement festlegbar ist.

Bei einer weiterhin bevorzugten Ausführungsform weist das innere Anlageelement und vorzugsweise auch das Verbindungselement eine vorzugsweise verschließbare Durchgangsöffnung auf, die zur Aufnahme eines im Hohlorgan liegenden Führungsdrahtes ausgebildet ist. Ein solcher Führungsdraht liegt bei einem chirurgischen oder interventionellen Eingriff normalerweise in dem Hohlorgan und insbesondere innerhalb eines in das Hohlorgan eingeführten rohrförmigen Instrumentes, insbesondere eines Katheters oder Trokars. Nach dem Eingriff kann das rohrförmige Instrument entfernt werden, so dass lediglich noch der Führungsdraht im Hohlorgan verbleibt und aus diesem herausragt. Auf das herausragende Stück des Führungsdrahtes können dann die einzelnen Teile des erfindungsgemäßen Verschlusssystems aufgeschoben werden, wobei der Draht dazu dient, die normalerweise nicht sichtbare Punktionsöffnung zum Einführen des inneren Anlageelementes zu finden. Die Durchgangsöffnung bzw. der von dieser gebildete Durchgangskanal ist vorzugsweise verschließbar ausgebildet, so dass er nach der Montage und nach dem Herausnehmen des Führungsdrahtes verschlossen werden kann. Ein Verschließen der Durchgangsöffnung kann beispielsweise durch Abquetschen des Ansatzes des Verbindungselementes vorgenommen werden, insbesondere im Zusammenhang mit einem Kürzen des Verbindungselementes zum Entfernen einer als Applikationshilfe dienenden Verlängerung. Einer solchen Ausführungsform mit einer Durchgangsöffnung insbesondere in Verbindung mit einem Führungsdraht ist vorzugsweise eine rohrförmige Applikationshilfe zugeordnet, die ebenfalls über den Führungsdraht geschoben werden kann.

Das Verbindungselement greift normalerweise im Zentrum des streifenförmig ausgebildeten inneren Anlageelementes an und kann das Zentrum des inneren Anlageelementes bilden. Ist das innere Anlageelement beispielsweise als Flachspirale ausgebildet, dann ist das Zentrum der innere Anfang der Spirale. Ist das innere Anlageelement mäanderförmig ausgebildet, dann liegt das Zentrum vorzugsweise in einem mittleren Streifenabschnitt. Bei spiraliger Ausbildung wird normalerweise das innere Ende der Spirale als Anfang zum Einführen durch die Punktionsöffnung gewählt. Es sind aber auch Ausführungsformen möglich, bei denen das äußere Ende als Anfang beim Einführen verwendbar ist. Bei mäanderförmig gebogenen Streifen kann das eine oder andere Ende als Anfang zum Einführen in das Hohlorgan benutzt werden.

Sämtliche Teile des erfindungsgemäßen Verschlusssystems bestehen vorzugsweise aus bioverträglichem Material. Die einzelnen Teile können resorbierbar oder nicht resorbierbar sein. Sind die wesentlichen Funktionsteile des Verschlusses nicht aus resorbierbarem Material gebildet, dann ist der Verschluss vorzugsweise ein wieder entfernbarer Verschluss. Im Gegensatz zum Stand der Technik, kann beim erfindungsgemäßen Verschluss das streifenförmige innere Anlageelement in gleicher Weise wieder durch die Punktionsöffnung herausgeführt werden, wie es eingeführt wurde. Die Punktionsöffnung steht dann für einen erneuten Eingriff wieder zur Verfügung.

Bei einer anderen Ausführungsform der Erfindung sind vorzugsweise sämtliche nach der Montage am Hohlorgan verbleibenden Teile des Verschlusses resorbierbar ausgebildet. Bevorzugt sind sämtliche Teile des Verschlusses aus einem resorbierbaren Kunststoffmaterial gebildet. Die Resorptionsdauer und mechanischen Eigenschaften können durch Wahl geeigneter Materialien vorbestimmt werden. Die Resorptionsdauer beträgt vorzugsweise in vivo 6 bis 24 Wochen. Die Resorption des Materials des Verschlusses kann parallel zur Wundheilung stattfinden, so dass die Punktionsöffnung in dem Maße zuwächst wie die Teile des Verschlusses resorbiert werden.

Als Material für die resorbierbaren Teile eignen sich besonders synthetische Polymere wie Polyglykolid, Polylactid, Poly-ε-Caprolacton, Polytri-methylencarbonat und Poly-p-dioxan. Es sind auch Copolymere aus Monomeren dieser Polymere geeignet.

Das erfindungsgemäße Verschlusssystem findet, wie anfangs bereits erwähnt, vorzugsweise Anwendung in der Angiologie, Kardiologie, interventionellen Radiologie, Neuroradiologie und/oder Chirurgie zum Verschließen eines eine Punktionsöffnung aufweisenden Hohlorgans, insbesondere eines Blutgefäßes.

Weitere Merkmale und Vorteile der Erfindung ergeben sich aus der nachfolgenden Beschreibung von bevorzugten Ausführungsformen der Erfindung in Verbindung mit den Unteransprüchen und der Zeichnung. Hierbei können die einzelnen Merkmale jeweils für sich oder in Kombination miteinander verwirklicht sein.

In der Zeichnung zeigen:
- Figur 1:: eine Ausführungsform des erfindungsgemäßen Punktionsverschlusses im montierten Zustand,
- Figur 2:: einen Querschnitt durch die Ausführungsform nach Figur 1,
- Figur 3:: die Ausführungsform nach den Figuren 1 und 2 im noch nicht montierten Zustand,
- Figur 4:: die Ausführungsform nach den Figuren 1 bis 3 während der Montage,
- Figur 5:: eine andere Ausführungsform in Draufsicht,
- Figur 6:: die Ausführungsform nach Figur 5 im Schnitt bei der Montage,
- Figur 7:: die Ausführungsform nach Figur 5 nach der Montage im Schnitt und
- Figur 8:: eine weitere Ausführungsform.

### Figurenbeschreibung

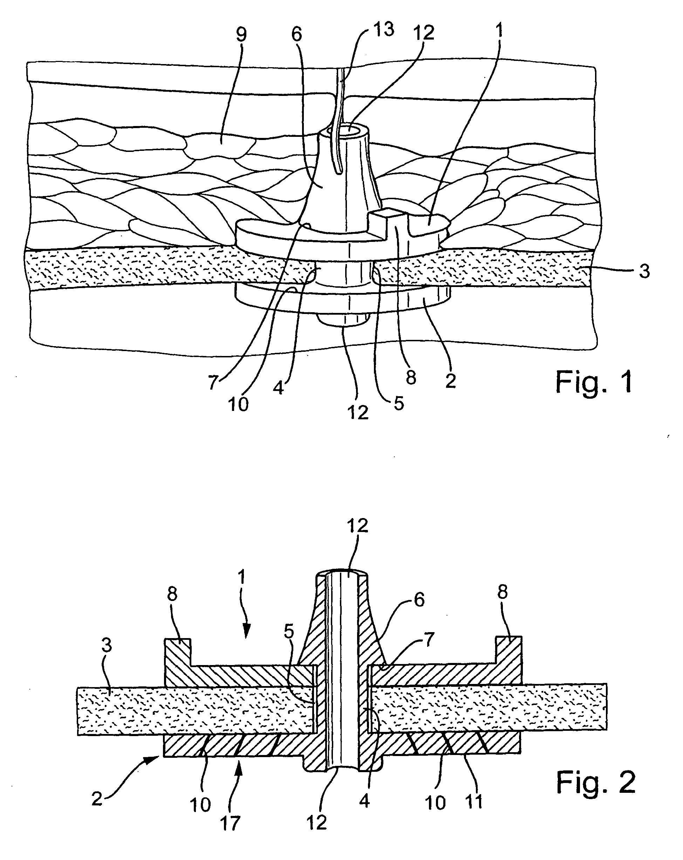

Bei der in Figur 1 und 2 dargestellten Ausführungsform der Erfindung ist ein Verschluss im Wesentlichen druckknopfartig ausgebildet und im montierten Gebrauchszustand dargestellt. Ein äußeres Anlageelement 1 weist die Form einer kreisrunden Lochscheibe auf. Ein zweites als Gegenstück dienendes Anlageelement 2 ist ebenfalls kreisförmig ausgebildet und hat im Wesentlichen den gleichen Durchmesser wie das Anlageelement 1. Anlageelement 1 liegt auf der Außenseite einer Arterienwandung 3 auf. Das Anlageelement 2 liegt auf der Innenseite der Arterienwandung. Die beiden Anlageelemente 1 und 2 sind über ein Verbindungselement 4 miteinander verbunden, das durch eine Punktionsöffnung 5 in der Arterienwandung 3 ragt. Das Verbindungselement 4 ist rohrförmig ausgebildet und unlösbar mit dem inneren Anlageelement 2 verbunden, beispielsweise einstückig mit diesem ausgebildet. Das Verbindungselement 4 durchdringt das äußere lochscheibenförmige Anlageelement 1 und weist an einem dieses Anlageelement überragenden Abschnitt ein Rastelement 6 auf, durch das die beiden Anlageelemente 1 und 2 gegeneinander verriegelt sind und dicht auf der Innen- und Außenseite der Arterienwandung gehalten werden. Das Rastelement 6 weist eine konische Verdickung am Verbindungselement 4 auf, die elastisch ausgebildet ist und die im Bereich des größten Durchmessers eine Hinterschneidung bzw. Schulter 7 aufweist. Das Anlageelement 1 ist zur Fixierung über die konische Erweiterung geschoben und hinter der Schulter 7 eingeschnappt. Das äußere Anlageelement 1 weist auf seiner von der Arterienwandung 3 abweisenden Außenseite zwei Nocken 8 auf, von denen in Figur 1 nur einer zu sehen ist. Der andere ist durch die über der Arterienwandung 3 liegende Haut oder Unterhaut 9 verdeckt. Die Nocken 8 dienen zum Ansetzen eines als Applikationshilfe dienenden Werkzeuges.

Das äußere Anlageelement 1 ist als massive Lochscheibe ausgebildet. Demgegenüber ist das innere Anlageelement 2 durch eine spiralförmig von innen nach außen verlaufende Trennlinie 10 unterteilt. Dadurch ist das innere Anlageelement 2 selbst spiralförmig ausgebildet, wobei die Gänge der flachen Spirale als flache Streifen 11 vorliegen, deren Breite in radialer Ausdehnung kleiner ist als die größte Längenausdehnung der Öffnung 5 in der Arterienwandung 3. Die spiralig verlaufende Trennlinie 10 verläuft vorzugsweise schräg zur Fläche des Anlageelements und zwar in der von dem ersten Anlageelement weg gerichteten Richtung nach außen. Dadurch findet eine Überlappung der Streifen 11 statt, was im Gebrauchszustand zu einer gegenseitigen Stabilisierung führt.

Die Durchgangsöffnung 12 im rohrförmigen Verbindungselement 4 ist zur Abdichtung verschlossen. Ein Faden 13, der am rohrförmigen Verbindungselement, insbesondere im konischen Bereich, befestigt ist, kann vorgesehen sein, um das rohrförmige Verbindungselement 4 durch die Öffnung des als Lochscheibe ausgebildeten äußeren Anlageelementes 1 hindurchzuziehen.

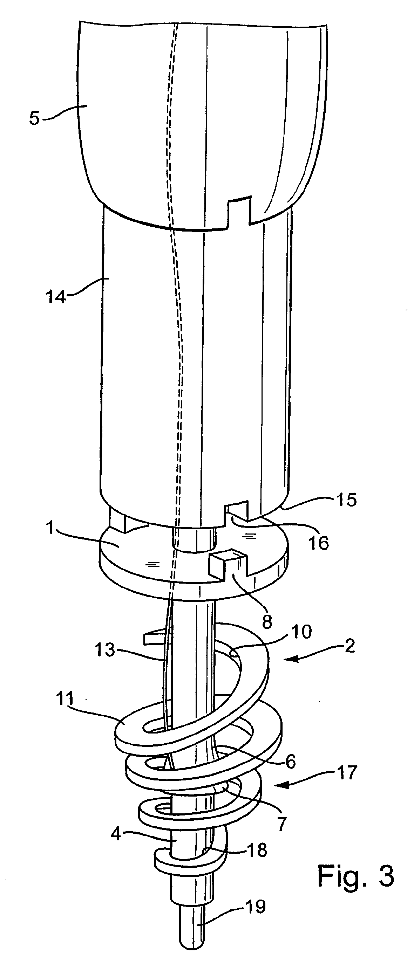

Figur 3 zeigt ein Verschlusssystem in Form der Elemente des Verschlusses nach den Figuren 1 und 2 und einem Werkzeug 14. Dieses Werkzeug ist rohrförmig ausgebildet und weist an seiner Stirnseite 15 zwei Ausnehmungen 16 auf, in die die Nocken 8 des äußeren Anlageelementes 1 passen. Das äußere Anlageelement 1 ist auf dem rohrförmigen Verbindungselement 4 in axialer Richtung verschiebbar, bei einer bevorzugten Ausführungsform jedoch drehfest mit diesem verbunden. Das Verbindungselement 4 ist bei dieser Ausführungsform vor und während der Montage länger als nach der Montage, wo es in gekürzter Form vorliegt. Hierzu kann eine Längsnut im Verbindungselement 4 vorgesehen sein, in die ein radialer nach innen gerichteter Vorsprung (nicht dargestellt) des ersten Verbindungselementes ragt. Durch Drehen des Drehwerkzeuges 14 kann somit eine Drehbewegung auf die Elemente des Verschlusses ausgeübt werden.

Figur 3 zeigt das spiralförmig ausgebildete innere Anlageelement 2 in entspanntem Zustand. Es weist die Form einer Wendelspirale 17 auf, bei der die einzelnen Gänge sowohl in axialer als auch in radialer Richtung gegeneinander versetzt sind. Die Spirale ist weiterhin als Flachspirale ausgebildet, d.h. in zusammengedrücktem Zustand liegen die einzelnen streifenförmigen Windungen unter Bildung des scheibenförmigen inneren Anlageelementes flächig nebeneinander. Wie bereits oben erwähnt, ist das innere Anlageelement 2 einstückig mit dem Verbindungselement 4 verbunden, beispielsweise gemeinsam durch Spritzguß hergestellt. Die Verbindung 18 ist jedoch vorzugsweise labil, so dass sich die einzelnen Windungen der Wendelspirale 17 je nach Bedarf an das rohrförmige Verbindungselement 4 anlegen können. Dadurch entspricht die maximale Dicke des Verschlusses beim Einführen durch die Öffnung 5 in der Arterienwand 3 der Summe aus der Breite des Streifens 11 und dem Durchmesser des Verbindungselementes 4, was wesentlich weniger ist als der Durchmesser des gesamten Anlageelementes 2. In Figur 3 ist weiterhin noch ein flexibler Führungsdraht 19 dargestellt, der durch die Öffnung 12 im Verbindungselement 4 und durch das Werkzeug 14 hindurchgeführt ist. Ein solcher Führungsdraht liegt normalerweise bei einem perkutanen, transluminalen Kathetereingriff in der Arterie. Für den erfindungsgemäßen Verschluss ist es vorgesehen, diesen Führungsdraht nach dem Eingriff in der Arterie zu belassen und ihn als Führung zur Zentrierung des Verschlusses zu verwenden, indem das rohrförmige Verbindungselement 4 über den Draht 19 geschoben und damit automatisch zur Öffnung 5 in der Arterie geführt wird, die normalerweise durch das Hautgewebe oder Unterhautgewebe 9 überdeckt und nicht so leicht zu finden ist.

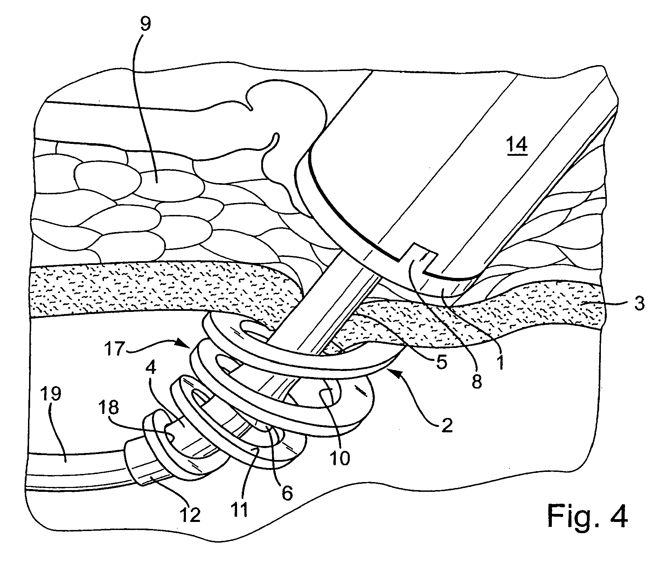

In Figur 4 ist das Anbringen des erfindungsgemäßen Verschlusses an einer Arterienwand dargestellt. Die Einführung der Verschlussteile in die Arterie erfolgt schräg, weil einerseits der Führungsdraht 19 schräg durch die Arterienwand 3 hindurch ragt und in der Arterie weiter verläuft und anderseits durch die schräge Einführung mehr Platz in der Arterie zur Verfügung steht. Die Einführung erfolgt durch Drehbewegung am Werkzeug 14, wodurch die Wendelspirale 17 durch die Wandung der Arterie hindurch eingeschraubt wird. Sobald die Wendelspirale vollständig durch die Arterienwand 3 hindurch geführt ist, wird das Verschlusselement 4 durch die Öffnung im äußeren Anlageelement angezogen, wobei gleichzeitig die Wendelspirale 17 unter Ausbildung des scheibenförmigen inneren Anlageelementes 2 komprimiert wird. Das Anziehen kann unmittelbar durch Zug am verlängerten Verschlusselement 4 erfolgen oder durch Zug am Faden 13. Ist die Verdickung bzw. das Rastelement 6 des Verbindungselementes 4 durch die Öffnung des äußeren Anlageelementes 1 hindurch geführt und die Rastverbindung eingeschnappt, dann wird der in Figur 1 dargestellte Zustand erreicht, wobei die beiden Anlageelemente 1 und 2 durch die Federwirkung der Spirale 17 federnd und abdichtend an der Arterienwandung 3 anliegen. Der überstehende und in den Figuren 3 und 4 dargestellte Teil des Verbindungselementes 4 kann nach Herausziehen des Führungsdrahtes 19 abgeschnitten und gleichzeitig abgequetscht werden, so dass ein dichter Verschluss vorliegt.

Die einzelnen Teile des erfindungsgemäßen Verschlusses bestehen vorzugsweise aus resorbierbaren Kunststoffen wie Polymeren und Copolymeren von α-Hydroxysäuren und dergleichen, wie Glykolid, Lactid, Trimethylencarbonat und Caprolacton. Durch geeignete Wahl der Bestandteile der Polymere kann die gewünschte Resorptionszeit eingestellt werden. Diese wird vorzugsweise so gewählt, dass die Resorption des Verschlusses zeitgleich mit der Zuheilung der Öffnung in der Arterienwand verläuft.

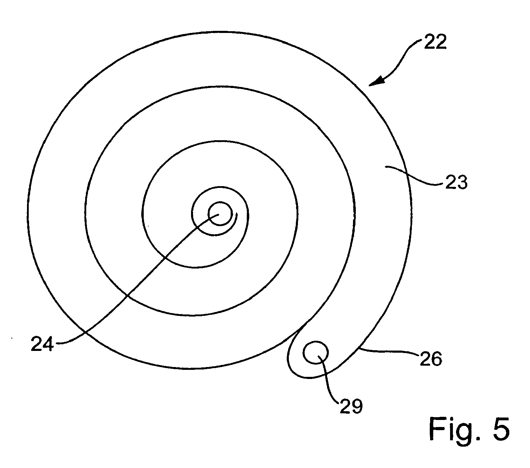

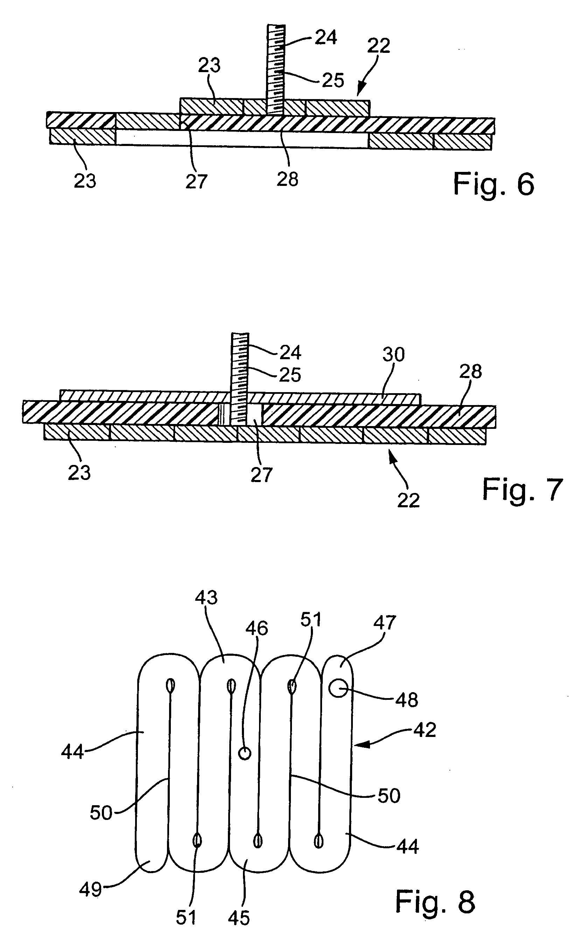

Bei der Ausführungsform nach den Figuren 5 bis 7 ist aus Vereinfachungsgründen nur ein inneres Anlageelement 22, das als Spirale 23 ausgebildet ist, und ein damit verbundenes stabförmiges Verbindungselement 24 dargestellt. Das Verbindungselement 24 greift im Zentrum der Spirale 23 an und ist massiv ausgebildet. Es kann oberhalb der Spirale mit einem Gewinde 25 versehen sein. Bei dieser Ausführungsform ist das freie Ende 26 der Spirale 23 dazu vorgesehen, als erstes durch eine Punktionsöffnung 27 einer Gefäßwand 28 eingeführt zu werden. Durch Drehung der Spirale 23 wird dann die gesamte Spirale beginnend von außen nach innen durch die Öffnung geführt. Dabei kann die Spirale mit Hilfe des stabförmigen Verbindungsstückes 24 gedreht werden. Auch hier wird das innere Anlageelement im Wesentlichen parallel zur Gefäßwandung in das Blutgefäß eingeführt, wie dies in Figur 6 schematisch dargestellt ist. Diese Ausführungsform ist in erster Linie geeignet für die Anwendung bei Punktionsöffnungen, die sichtbar sind oder mit Hilfsmittel sichtbar gemacht werden können. Dann kann das freie Ende 26 unmittelbar in die Öffnung 27 in der Gefäßwandung eingeführt werden. Ist die Öffnung 27 nicht sichtbar, dann kann wiederum ein Führungsdraht vorgesehen sein, der durch die Öffnung 27 in der Gefäßwandung ragt. Am freien Ende 26 der Wendelspirale 23 kann ein entsprechendes Führungsloch 29 vorgesehen sein, das durch den liegenden Führungsdraht geführt wird. Mit Hilfe des Führungsdrahtes wird somit die Öffnung 27 in der Gefäßwandung 28 gefunden. Nach Einführen des freien Endes 26 kann dann der Führungsdraht entfernt und der Rest der Spirale 23 durch Drehung eingedreht werden. Als äußeres Anlageelement kann bei dieser Ausführungsform eine Lochscheibe 30 mit Innengewinde vorgesehen sein, die über das stabförmige Verbindungselement 24 geschoben und durch Drehung auf dem Gewinde 25 bis zur Anlage an die äußere Gefäßwand eingedreht werden kann (vgl. Figur 7).

Bei der Ausführungsform nach Figur 8 ist ein inneres Anlageelement 42 vorgesehen, das wiederum streifenförmig ausgebildet ist, aber dieses Mal in Form eines flachen Mäanders 43 mit aneinander liegenden Streifenabschnitten. Es ist eine ungerade Anzahl von Streifenabschnitten 44 vorgesehen. In diesem Falle sind es sieben. In der Mitte des mittleren Streifenabschnittes 45 befindet sich die Ansatzstelle für ein Verbindungselement 46, das in diesem Falle als flexibler Faden bzw. Draht ausgebildet ist. Auch bei dieser Ausführungsform beginnt die Einführung des inneren Anlageelementes 42 durch eine Öffnung in einer Gefäßwandung an einem freien Ende 47. Auch hier kann das freie Ende mit einer Führungsöffnung 48 versehen sein, durch das ein ohnehin vorgesehener Führungsdraht geführt werden kann. Nach Einführung des freien Endes 47 in die Öffnung des Gefäßes wird der Führungsdraht entfernt und das mäanderförmige Anlageelement 42 abschnittsweise durch die Öffnung in der Gefäßwand geschoben bis das andere freie Ende 49 des Mäanders im Gefäß verschwunden ist. Die Streifenabschnitte 44 und 45 sind durch schmale Einschnitte 50 voneinander getrennt. Lediglich an den Enden der Trennlinien finden sich schmale Durchbrüche 51, die das Einführen des Streifens durch die Punktionsöffnung erleichtern. Durch Ziehen am flexiblen Verbindungselement 46 wird das innere Anlageelement 42 zentriert. Die Befestigung kann dann durch Anbringen eines äußeren Anlageelementes oder durch andere geeignete Maßnahmen erfolgen.

## Patentansprüche

1. Punktionsverschluss zum Verschließen eines eine Punktionsöffnung in einer Wandung aufweisenden Hohlorgans, insbesondere eines Blutgefäßes, umfassend ein inneres Anlageelement (2; 22; 42;) zur Anlage an die intraluminale Seite der Wandung des Hohlorgans und ein mit dem inneren Anlageelement verbundenes Verbindungselement (4; 24; 46), das im an der Wandung montierten Zustand des Verschlusses durch die Punktionsöffnung (5; 27) hindurch ragt, wobei das innere Anlageelement (2; 22; 42) eine Flächenausdehnung besitzt, die größer als die Punktionsöffnung ist, **dadurch gekennzeichnet, dass** das innere Anlageelement (2; 22; 42) als zusammenhängender bogenförmig verlaufender Streifen (17, 23; 43) ausgebildet ist, wobei Streifenabschnitte (11; 23; 45) im montierten Zustand flach nebeneinander liegen und die Streifenabschnitte eine Breite haben, die kleiner ist als die größte Länge der Punktionsöffnung (5; 27), und das innere Anlageelement (2; 22; 42) als Streifen (17; 23; 43) durch die Punktionsöffnung im Wesentlichen parallel zur Wandung (3; 28) des Hohlorgans in dieses einführbar ist.

2. Punktionsverschluss nachAnspruch 1, **dadurch gekennzeichnet, dass** der Streifen zumindest im montierten Zustand flach ist und in der Fläche des inneren Anlageelementes (2; 22; 42) bogenförmig verläuft.

3. Punktionsverschluss nach Anspruch 1 oder 2, **dadurch gekennzeichnet, dass** der Streifen (17; 23; 43) derart bogenförmig ausgebildet ist, dass sich benachbarte Streifenabschnitte (11; 23, 45) bilden.

4. Punktionsverschluss nach einem der vorhergehenden Ansprüche, **dadurch gekennzeichnet, dass** das innere Anlageelement (2; 22; 42) zumindest im montierten Zustand scheibenförmig ausgebildet ist.

5. Punktionsverschluss nach einem der vorhergehenden Ansprüche, **dadurch gekennzeichnet, dass** der Streifen (17; 23; 43) spiralig gebogen verläuft.

6. Punktionsverschluss nach einem vorhergehenden Ansprüche, **dadurch gekennzeichnet, dass** der Streifen (23) in Form einer flächigen Spirale vorliegt, oder als spiralige Wendel ausgebildet ist.

7. Punktionsverschluss nach einem der vorhergehenden Ansprüche, **dadurch gekennzeichnet, dass** der Streifen (43) im Wesentlichen schlangenförmig bzw. mäanderförmig gebogen ist.

8. Punktionsverschluss nach einem der vorhergehenden Ansprüche, **dadurch gekennzeichnet, dass** benachbarte Streifenabschnitte (11; 23, 45) einen Abstand haben oder in einen solchen bringbar sind, der zumindest der Wandungsstärke des Hohlorgans entspricht.

9. Punktionsverschluss nach einem der vorhergehenden Ansprüche, **dadurch gekennzeichnet, dass** ein Abstand zwischen benachbarten Streifenabschnitten (11; 23, 45) durch elastische Verschiebung der Streifenabschnitte schräg, vorzugsweise senkrecht, zur Fläche des inneren Anlageelementes (2; 22; 42) erzeugbar oder erweiterbar ist.

10. Punktionsverschluss nach einem der vorhergehenden Ansprüche, **dadurch gekennzeichnet, dass** benachbarte Streifenabschnitte (11; 23) einen Abstand haben, der durch elastische Kompression verkleinerbar oder beseitigbar ist.

11. Punktionsverschluss nach einem der vorhergehenden Ansprüche, **dadurch gekennzeichnet, dass** sich benachbarte Streifenabschnitte (11) im montierten Zustand gegenseitig überlappen.

12. Punktionsverschluss nach einem der vorhergehenden Ansprüche, **dadurch gekennzeichnet, dass** benachbarte Streifenabschnitte (11; 23; 45) im montierten Zustand eine im Wesentlichen geschlossene Fläche bilden.

13. Punktionsverschluss nach einem der vorhergehenden Ansprüche, **dadurch gekennzeichnet, dass** dem inneren Anlageelement (2; 22; 42) ein äußeres Gegenlager (1; 30) zugeordnet ist, insbesondere ein äußeres Anlageelement zur Anlage an die extraluminale Seite des Hohlorgans, wobei das Gegenlager, insbesondere das äußere Anlageelement (1; 30), auf dem Verbindungselement (4; 24) festlegbar ist.

14. Punktionsverschluss nach einem der vorhergehenden Ansprüche, **dadurch gekennzeichnet, dass** das innere Anlageelement (2; 22; 42) und vorzugsweise auch das Verbindungselement (4) eine vorzugsweise verschließbare Durchgangsöffnung (12; 29; 48) aufweist, die zur Aufnahme eines im Hohlorgan liegenden Führungselementes (19) ausgebildet ist.

15. Punktionsverschluss nach einem der vorhergehenden Ansprüche, **dadurch gekennzeichnet, dass** dem Verschluss, insbesondere dem inneren Anlageelement (2; 22; 42) eine Applikationshilfe (14; 24; 46) zugeordnet ist, die mit dem Verschluss, insbesondere dem inneren Anlageelement, zugefest verbindbar oder verbunden ist.

## Claims

1. A puncture seal for sealing a hollow organ having a puncture opening in a wall, in particular a blood vessel, said puncture seal comprising an inner bearing element (2; 22; 42) for bearing on the intraluminal side of the wall of the hollow organ, and a connection element (4; 24; 46) which is connected to the inner bearing element and which, when the seal is fitted on the wall, protrudes through the puncture opening (5; 27), wherein the inner bearing element (2; 22; 42) has a surface area greater than the puncture opening, **characterized in that** the inner bearing element (2; 22; 42) is designed as a continuous strip (17; 23; 43) extending in an arc shape, wherein strip sections (11; 23; 45) lie flat alongside one another in the fitted state, and the strip sections have a width that is smaller than the greatest length of the puncture opening (5; 27), and the inner bearing element (2; 22; 42) is insertable into the hollow organ through the puncture opening as a strip (17; 23; 43) and essentially parallel to the wall (3; 28) of the hollow organ.

2. The puncture seal according to claim 1, **characterized in that** the strip, at least in the fitted state, is flat and extends in an arc shape in the surface of the inner bearing element (2; 22; 42).

3. The puncture seal according to claim 1 or 2, **characterized in that** the strip (17; 23; 43) is configured in an arc shape in such a way that adjacent strip sections (11; 23; 45) are produced.

4. The puncture seal according to any one of the preceding claims, **characterized in that** the inner bearing element (2; 22; 42), at least in the fitted state, has a disk-shaped configuration.

5. The puncture seal according to any one of the preceding claims, **characterized in that** the strip (17; 23; 43) curves in a spiral shape.

6. The puncture seal according to any one of the preceding claims, **characterized in that** the strip (23) is in the form of a flat spiral, or is designed as a helical coil.

7. The puncture seal according to any one of the preceding claims, **characterized in that** the strip (43) curves in a substantially serpentine or meandering shape.

8. The puncture seal according to any one of the preceding claims, **characterized in that** adjacent strip sections (11; 23; 45) have a spacing, or can be brought into a spacing, that corresponds at least to the wall thickness of the hollow organ.

9. The puncture seal according to any one of the preceding claims, **characterized in that** a spacing between adjacent strip sections (11; 23; 45) can be generated or widened by elastic displacement of the strip sections obliquely, preferably perpendicularly, with respect to the surface of the inner bearing element (2; 22; 42).

10. The puncture seal according to any one of the preceding claims, **characterized in that** adjacent strip sections (11; 23) have a spacing that can be made smaller or can be eliminated by elastic compression.

11. The puncture seal according to any one of the preceding claims, **characterized in that** adjacent strip sections (11) mutually overlap in the fitted state.

12. The puncture seal according to any one of the preceding claims, **characterized in that** adjacent strip sections (11; 23; 45) form a substantially closed surface in the fitted state.

13. The puncture seal according to any one of the preceding claims, **characterized in that** the inner bearing element (2; 22; 42) is assigned an outer abutment (1; 30), in particular an outer bearing element for bearing on the extraluminal side of the hollow organ, wherein the outer abutment, in particular the outer bearing element (1; 30), can be secured on the connection element (4; 24).

14. The puncture seal according to any one of the preceding claims, **characterized in that** the inner bearing element (2; 22; 42) and preferably also the connection element (4) have a preferably closable through-opening (12; 29; 48) that is designed to receive a guide element (19) lying in the hollow organ.

15. The puncture seal according to any one of the preceding claims, **characterized in that** the seal, in particular the inner bearing element (2; 22; 42), is assigned an application aid (14; 24; 46) which is connected or can be connected tautly to the seal, in particular to the inner bearing element.

## Revendications

1. Fermeture de ponction destinée à fermer un organe creux présentant une ouverture de ponction dans une paroi, en particulier un vaisseau sanguin, comprenant un élément d'appui intérieur (2; 22; 42) pour l'application sur le côté intraluminal de la paroi de l'organe creux et un élément de liaison (4; 24; 46) relié à l'élément d'appui intérieur, qui pénètre à travers l'ouverture de ponction (5; 27) dans l'état monté de la fermeture sur la paroi, dans laquelle l'élément d'appui intérieur (2; 22; 42) possède une extension superficielle qui est plus grande que l'ouverture de ponction, **caractérisée en ce que** l'élément d'appui intérieur (2; 22; 42) est réalisé sous forme de ruban continu s'étendant en forme d'arc (17, 23; 43), dans laquelle des parties de ruban (11; 23; 45) sont appliquées à plat l'une à côté de l'autre dans l'état monté et les parties de ruban ont une largeur, qui est plus petite que la plus grande longueur de l'ouverture de ponction (5; 27) et l'élément d'appui intérieur (2; 22; 42) peut être introduit dans l'organe creux, en tant que ruban (17; 23; 43) à travers l'ouverture de ponction, essentiellement parallèlement à la paroi (3; 28) de celui-ci.

2. Fermeture de ponction selon la revendication 1, **caractérisée en ce que** le ruban est plat au moins dans l'état monté et il s'étend en forme d'arc dans la face de l'élément d'appui intérieur (2; 22; 42).

3. Fermeture de ponction selon la revendication 1 ou 2, **caractérisée en ce que** le ruban (17; 23; 43) est configuré en forme d'arc, de telle manière qu'il se forme des parties de ruban voisines (11; 23, 45).

4. Fermeture de ponction selon l'une quelconque des revendications précédentes, **caractérisée en ce que** l'élément d'appui intérieur (2; 22; 42) est configuré en forme de disque au moins dans l'état monté.

5. Fermeture de ponction selon l'une quelconque des revendications précédentes, **caractérisée en ce que** le ruban (17; 23; 43) s'étend avec une courbure en spirale.

6. Fermeture de ponction selon l'une quelconque des revendications précédentes, **caractérisée en ce que** le ruban (23) se présente sous la forme d'une spirale plate, ou est réalisé en forme d'hélice spirale.

7. Fermeture de ponction selon l'une quelconque des revendications précédentes, **caractérisée en ce que** le ruban (43) est courbé essentiellement en forme de serpentin ou de méandres.

8. Fermeture de ponction selon l'une quelconque des revendications précédentes, **caractérisée en ce que** des parties de ruban voisines (11; 23, 45) ont une distance ou peuvent être y être amenées, qui correspond au moins à l'épaisseur de paroi de l'organe creux.

9. Fermeture de ponction selon l'une quelconque des revendications précédentes, **caractérisée en ce qu'**une distance entre des parties de ruban voisines (11; 23, 45) peuvent être produites ou agrandies par un déplacement élastique des parties de ruban en oblique, de préférence perpendiculairement à la face de l'élément d'appui intérieur (2; 22; 42).

10. Fermeture de ponction selon l'une quelconque des revendications précédentes, **caractérisée en ce que** des parties de ruban voisines (11; 23) ont une distance qui peut être réduite ou supprimée par une compression élastique.

11. Fermeture de ponction selon l'une quelconque des revendications précédentes, **caractérisée en ce que** des parties de ruban voisines (11) se chevauchent mutuellement dans l'état monté.

12. Fermeture de ponction selon l'une quelconque des revendications précédentes, **caractérisée en ce que** des parties de ruban voisines (11; 23; 45) forment une face essentiellement fermée dans l'état monté.

13. Fermeture de ponction selon l'une quelconque des revendications précédentes, **caractérisée en ce qu'**une butée extérieure (1; 30) est associée à l'élément d'appui intérieur (2; 22; 42), en particulier un élément d'appui extérieur pour l'appui sur le côté extraluminal de l'organe creux, dans laquelle la butée, en particulier l'appui extérieur (1; 30), peut être fixé(e) sur l'élément de liaison (4; 24).

14. Fermeture de ponction selon l'une quelconque des revendications précédentes, **caractérisée en ce que** l'élément d'appui intérieur (2; 22; 42) et de préférence aussi l'élément de liaison (4) présente une ouverture de passage (12; 29; 48) pouvant de préférence être fermée, qui est configurée pour recevoir un élément de guidage (19) situé dans l'organe creux.

15. Fermeture de ponction selon l'une quelconque des revendications précédentes, **caractérisée en ce qu'**un accessoire d'application (14; 24; 46) est associé à la fermeture, en particulier à l'élément d'appui intérieur (2; 22; 42), qui peut être ou qui est relié de façon résistant à la traction à la fermeture, en particulier à l'élément d'appui intérieur.
